# EUROPEAN PATENT APPLICATION

(11) **EP 1 356 838 A1**
(43) Date of publication of application: **29.10.2003**
(21) Application number: 02076657.2
(22) Date of filing: 25.04.2002
(51) Int. Cl.: A61M 5/32, A61M 5/31

(54) **Self-locking disposable safety syringe having a retractable needle hub**

(71) Applicant: Hsu, Chi-Hsuan, Taipei (TW)
(72) Inventor: Hsu, Chi-Hsuan, Taipei (TW)
(74) Representative: Vernout, Robert

(57) **Abstract**

A self-locking disposable safety syringe includes a hollow barrel (10) having a front open end, a rear open end and a flange (12) formed on an inner face of the barrel (10), a plunger (20) extendable into the barrel (10) from the rear open end, a needle hub connecting assembly (30) selectively movable in the barrel (10) for securely engaging with a needle hub (40) which is inserted into the barrel (10) from the front end, the needle hub connecting assembly (30) having an annular ring (33) expandable in the barrel, and a press movably received in the barrel (10) and securely connected to the needle hub connecting assembly (30) to selectively expand the annular ring (33) so as to position the needle hub connecting assembly (30) in the barrel (10).

## Description

### 1. Field of the Invention

The present invention relates to a syringe and more particularly to a self-locking disposable safety syringe. The syringe has a C-shaped ring expandably received in a needle hub and a press with a key extending into a keyway in the needle hub connector to expand the ring such that the forward movement of the plunger to the needle is secured due to the abutment of the expanded ring to an inwardly formed flange on an inner face of the barrel of the syringe and a backward movement of the plunger is able to release the expansion of the ring in the needle hub connector so as to allow the needle hub connector as well as the needle hub to be pulled backward into the barrel for safety.

### 2. Description of Related Art

Healthcare workers routinely have the risk of contracting infective diseases through accidental exposure to contaminated medical waste. One of the largest exposure risks to healthcare workers and handlers of the medical waste is from accidental pricking by used syringe needles. It is reported that odds of a healthcare worker contracting human immune deficiency virus (HIV) alone through accidental pricking are one in 300. The odds of healthcare workers contracting other serious diseases are even greater.

Therefore a current disposable syringe having a retractable needle is designed to solve the accidental pricking problem. The disposable syringe typically has a retractable needle hub with a needle securely mounted on the needle hub, which is able to be captured by a plunger. By pulling the plunger backward into the barrel, the needle is fully retracted inside a barrel, thus the contaminated syringe is able to be discarded safely without the risk of pricking someone.

However, the disposable syringe having the retractable needle is able to solve the accidental pricking problem only under an ideal situation. Further problems may still arise in practical use, for example, when retracting the needle, the connection between the needle and the plunger is not secure enough, and may undesirably detach before the needle is fully retracted inside the barrel. The undesirable detachment of the plunger to the needle may cause a health risk to the user due to the chance of accidental pricking.

Accordingly, the present invention tends to provide a self-locking disposable safety syringe to mitigate or obviate the aforementioned problems.

An objective of the present invention is to provide a self-locking disposable safety syringe to prevent accidental pricking, scratching or other harmful exposure to healthcare workers. The syringe has a barrel, a needle hub connector assembled at a front end of the barrel, a needle hub fitted securely engaged with the needle hub connector, a C-shaped ring expandably received in the needle hub connector and a press with a key inserted into a keyway in the needle hub connector to expand the ring so as to secure the connection between the needle hub connector and the barrel such that when the plunger is moving toward the needle hub connector, a flange inwardly formed on an inner face of the barrel is able to stop the needle hub connector and the press to move further into the barrel. When the plunger is moving away from the needle hub connector, the key leaves the keyway in the needle hub connector so as to release the expansion of the ring. Thereafter, the backward movement of the plunger inside the barrel will drive the needle hub connector together with the needle hub to move into the barrel to avoid accidental pricking to paramedics.

Another objective of the present invention is that a connection ring is provided on top of the press, which is to be inserted into a recess in a distal end of the plunger so that when the plunger is moving backward into the barrel, the plunger is able to drive the press and the needle hub connector to move along therewith.

Other objectives, advantages and novel features of the invention will become more apparent from the following detailed description when taken in conjunction with the accompanying drawings.

### IN THE DRAWINGS

Fig. 1 is a perspective view of the self-locking disposable safety syringe in accordance with the present invention;
Fig. 2 is an exploded perspective view of the syringe in Fig. 1;
Fig. 3 is a schematic view showing the connection between the press, the C-shaped ring and the needle hub connector, wherein the press is inserted into the keyway in the needle hub connector;
Fig. 4 is a schematic view showing the connection between the press, the ring and the needle hub connector, wherein the key of the press is inserted into the keyway of the needle hub connector;
Fig. 5 is a cross sectional view showing the relationship among the press, the ring, the needle hub connector and the needle hub in assembly; and
Fig. 6 is an enlarged cross sectional view showing the expansion of the ring is release due to the upward movement of the press caused by the upward movement of the plunger.

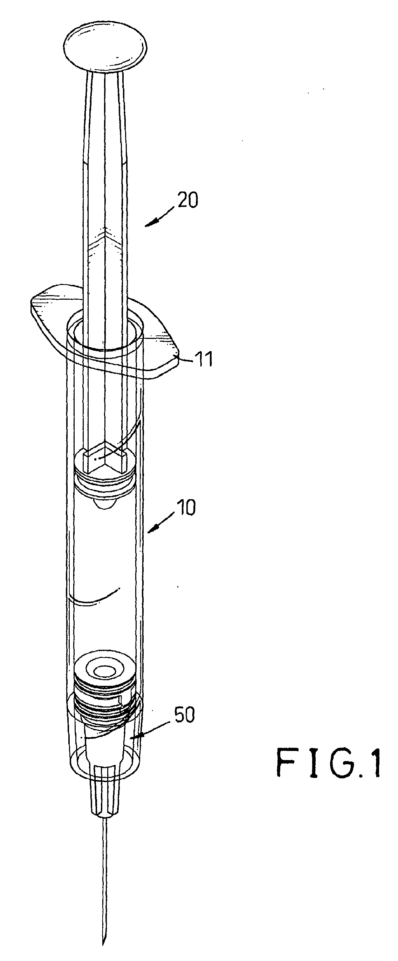

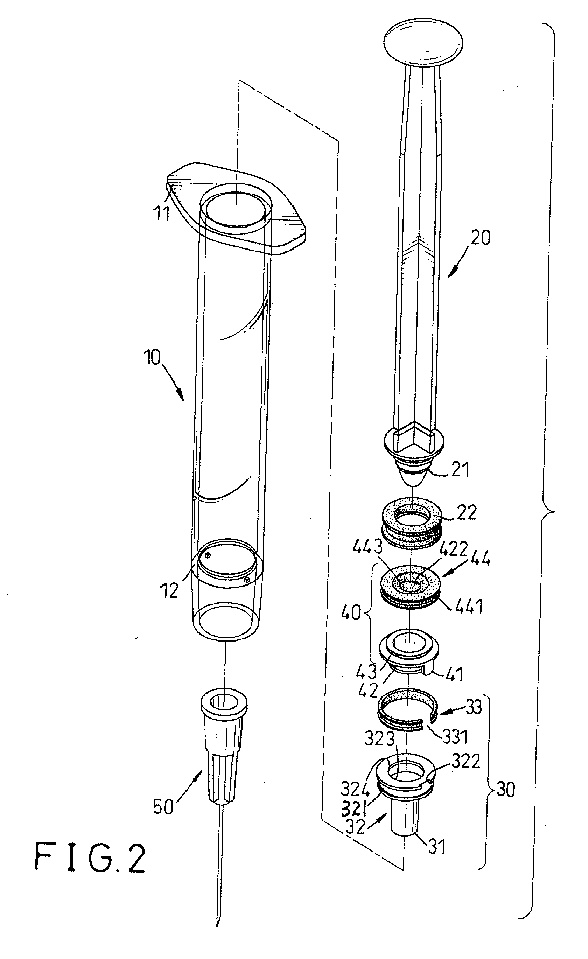

With reference to Figs. 1 and 2, a self-locking disposable safety syringe in accordance with the present invention includes a barrel (10), a plunger (20), a needle hub connecting assembly (30), an engaging device and a needle hub (50).

The barrel (10) is a hollow cylinder having a front open end and a rear open end. A finger flange (11) is integrally formed at and extending laterally from the rear open end. A flange (12) is formed on an inner face of the hollow cylindrical barrel (10) and a stop (13) is formed above the flange (12) on the inner face of the barrel (10).

The needle hub connecting assembly (30) is received in the barrel (10) and is composed of a needle hub connector and a C-shaped ring (33). The needle hub connector has a cylindrical extension (31) extending out from a bottom face of an annular seat (32). The annular seat (32) has an annular indent (321) defined in a mediate portion of the annular seat (32) and a keyway (322) defined in a top face of the annular seat (32) to communicate with the keyway (321). The C-shaped ring (33) has a gap (331) corresponding to the keyway (322) and is received in the indent (321). That is, after the ring (33) is received in the indent (321), the gap (331) coincides with the keyway (322). The annular seat (32) further has a blind hole (323) extending inwardly from the top face of the annular seat (32) and a lip (324) extending toward a center of the blind hole (323).

The press (40) has a key (41) formed to correspond to the keyway (322), a hook (42) formed on a bottom face of the press (40) to correspond to the lip (324) and a neck (43) formed on a top portion of the press (40). An annular first seal (44) has a groove (441) defined in a mediate portion of the first seal (44), a through hole (442) defined in a central portion of the seal (44) and a tongue (443) extending toward the center of the through hole (442).

The plunger (20) has an annular recess (21) adjacent a distal end thereof. A second seal (22) is mounted around the plunger (20) to ensure that after the plunger (20) is inserted into the barrel (10), the connection therebetween is a fluid-tight connection.

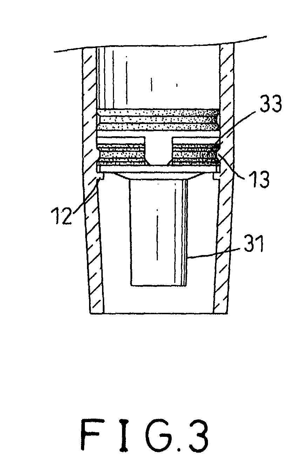

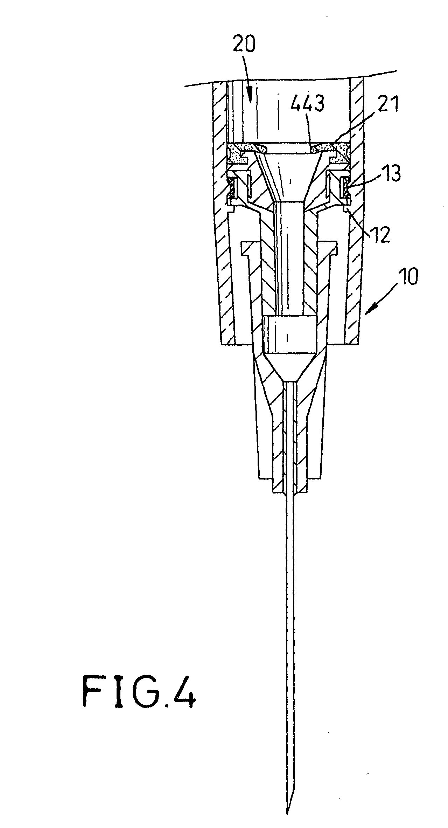

With reference to Figs. 3 and 4 and still taking Fig. 2 for reference, when the syringe of the present invention is to be assembled, the needle hub connector with the C-shaped ring (33) received in the indent (321) is first inserted into the hollow barrel (10). Due to the formation of the flange (12) and the annular seat (32) being tapered toward the cylindrical extension (31), the needle hub connector is retained in the barrel (10). It is to be noted that the free end of the key (41) is conical and has a width slightly larger than a width of the keyway (322) yet larger than a width of the gap (331), such that when the key (41) is inserted into the corresponding keyway (322) and the gap (331), the ring (33) is expanded. Before the ring (33) is expanded, it is noted from Fig. 3 that the ring (33) has a diameter smaller than an inner diameter of the stop (13) of the barrel (10) such that there is still room left between the ring (33) and the stop (13). However, after the expansion of the ring (33), the room is no longer available between the ring (33) and the stop (13).

After the assembly of the foregoing elements, the needle hub (50) is securely engaged with the needle hub connecting assembly (30), which is conventional in the art and the technique used is well known in the art. Therefore, the following detailed description contains no more information about how the needle hub (50) is connected to the needle hub connecting assembly (30).

Because of the expansion of the ring (33) in the barrel (10), the needle hub connecting assembly (30) and the press (40) are able to be securely retained in the barrel (10) when the needle hub (50) together with a needle (51) securely connected thereto engages with the needle hub connecting assembly (30), thus the needle hub connecting assembly (30) will not move. Thereafter, the user inserts the plunger (20) with the second seal (22) mounted therearound into the barrel (10).

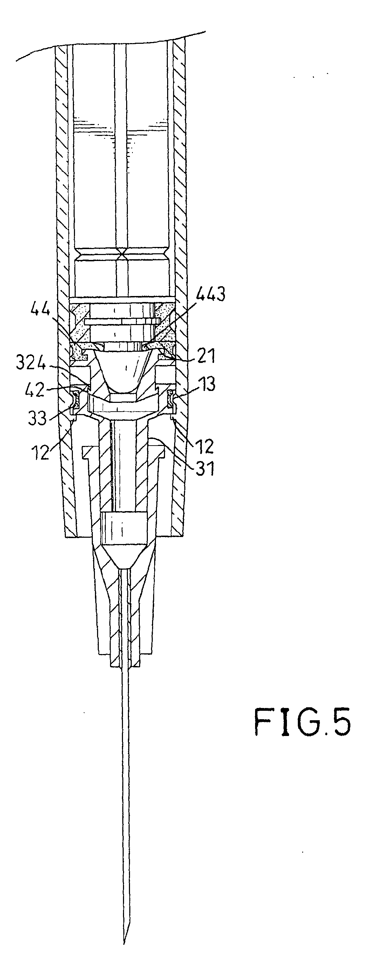

With reference to Fig. 5, the cross sectional view shows that after the plunger (20) is inserted into the barrel (10) and engages with the press (40), the tongue (443) is received in the recess (21) so that when the user wants to dispose the syringe of the present invention, the user just pulls the plunger (20) backward into the barrel (10). It is also noted that before the user inserts the plunger (20) into the barrel (10), the hook (42) of the press (40) is not in engagement with the lip (324).

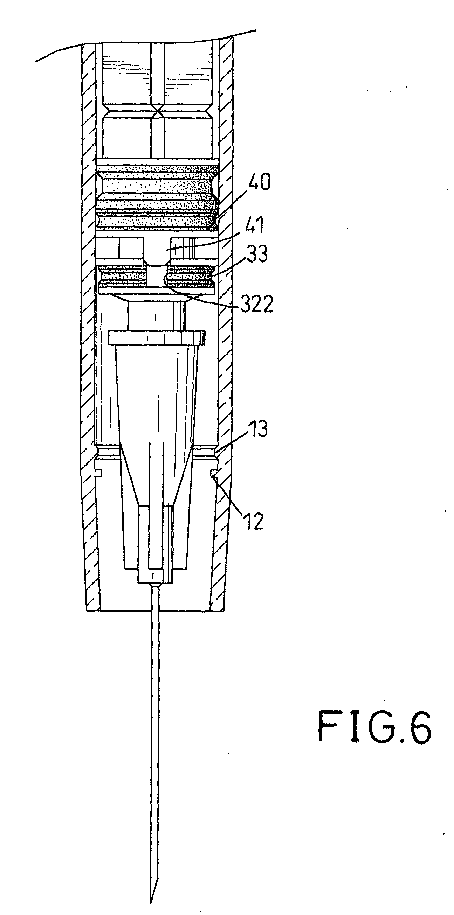

With reference to Figs. 5 and 6 and still taking Fig. 2 for reference, while the plunger (20) is moving inside the barrel (10), because the tongue (443) is received in the recess (21) of the plunger (20), the first seal (44), which is securely engaged with the press (40), will be pulled inward the barrel (10). When the press (40) is also moved due to the movement of the plunger (20), the key (41) leaves the keyway (322) of the needle hub connector, which releases the expansion of the ring (33). Furthermore, when the hook (42) engages with the lip (324) of the annular seat (32) of the needle hub connector, the upward movement of the plunger (20) will drive the needle hub connector to move along with the plunger (20).

After the needle hub connecting assembly (30), the press (40) as well as the needle hub (50) are all pulled into the barrel (10), the user is able to use the existing technology to break the connection of the plunger (20) to the needle hub connecting assembly (30). Thus the needle hub (50) together with the needle (51) is received in the barrel (10) and accidental pricking or scratching to paramedic personnel is reduced, as well as the total length of the syringe being reduced for convenient disposal.

While this invention has been particularly shown and described with references to the preferred embodiment thereof, it will be understood by those skilled in the art that various changes in form and details may be made therein without departing from the scope of the invention encompassed by the appended claims.

## Claims

1. A self-locking disposable safety syringe comprising:
a hollow barrel having a front open end, a rear open end, a flange formed on an inner face of the barrel and a stop formed above the flange;
a plunger extendable into the barrel from the rear open end;
a needle hub connecting assembly stopped by the flange and selectively movable in the barrel for securely engaging with a needle hub which is inserted into the barrel from the front end, the needle hub connecting assembly having an annular ring expandable in the barrel so as to engage with the stop; and
a press movably received in the barrel and securely connected to the needle hub connecting assembly to selectively expand the annular ring so as to position the needle hub connecting assembly in the barrel,
whereby when the annular ring is expanded, the needle hub connecting assembly is fixed in the barrel and when expansion of the annular ring is released, the needle hub connecting assembly is able to be pulled backward into the barrel by the plunger with the assistance of the press.

2. The syringe as claimed in claim 1, wherein the press has a hook formed on a bottom end thereof to correspond to a lip formed inside a blind hole in a center of the needle hub connecting assembly so that after the presser is engaged with the needle hub connecting assembly, an engagement between the hook and the lip ensures that the needle hub connecting assembly is moved together with the press when pulled backward into the barrel by the plunger.

3. The syringe as claimed in claim 2, wherein the press has a key formed to correspond to a keyway in the needle hub connecting assembly.

4. The syringe as claimed in claim 3, wherein the annular ring is received in an indent of the needle hub connecting assembly and has a gap corresponding to the keyway, wherein the gap is provided with a width smaller than a width of the key such that the annular ring is expanded when the key is inserted into the corresponding keyway.

5. The syringe as claimed in claim 1, wherein the plunger has a recess defined in a bottom end of the plunger, the press has a first seal mounted on top of the press and having a tongue extending toward a center of the first seal to correspond to the recess of the plunger so that when the first seal is received in the corresponding recess, the press is able to be driven by the plunger.

6. The syringe as claimed in claim 2, wherein the plunger has a recess defined in a bottom end of the plunger, the press has a first seal mounted on top of the press and having a tongue extending toward a center of the first seal to correspond to the recess of the plunger so that when the first seal is received in the corresponding recess, the press is able to be driven by the plunger.

7. The syringe as claimed in claim 4, wherein the plunger has a recess defined in a bottom end of the plunger, the press has a first seal mounted on top of the press and having a tongue extending toward a center of the first seal to correspond to the recess of the plunger so that when the first seal is received in the corresponding recess, the press is able to be driven by the plunger.

8. The syringe as claimed in claim 1, wherein the barrel has a stop formed below the flange on the inner face of the barrel to stop travel of the needle hub connecting assembly.

9. The syringe as claimed in claim 7, wherein the barrel has a stop formed below the flange on the inner face of the barrel to stop travel of the needle hub connecting assembly.

10. The syringe as claimed in claim 1, wherein the needle hub connecting assembly is composed of an annular seat and a cylindrical extension formed under the annular seat.

11. The syringe as claimed in claim 4, wherein the indent is defined in the annular seat and the keyway is defined in a top portion of the annular seat.

12. The syringe as claimed in claim 7, wherein the indent is defined in the annular seat and the keyway is defined in a top portion of the annular seat.

13. The syringe as claimed in claim 9, wherein the indent is defined in the annular seat and the keyway is defined in a top portion of the annular seat.
